# EUROPEAN PATENT APPLICATION

(11) **EP 4 803 902 A2**
(43) Date of publication of application: **09.09.2026**
(21) Application number: 26192000.3
(22) Date of filing: 30.09.2022
(51) Int. Cl.: G01N 33/542

(54) **IMPROVEMENTS IN OR RELATING TO A DEVICE FOR IMAGING**

(30) Priority: 01.10.2021 GB 202114087; 01.10.2021 GB 202114090
(62) Document number(s) of the earlier application(s) in accordance with Art. 76 EPC: 22786083.0 / 4 409 227
(71) Applicant: Panacea Diagnostics Ltd, London, WC2H 9JQ (GB)
(72) Inventor: SMITH, Callum, Robertson, 2100 København Ø (DK); Klug, David. R, London, WC2H 9JQ (GB); WORKUM, Stefan, Leo, Van, London, WC2H 9JQ (GB); TUREK, Vladimir, Alexander, London, WC2H 9JQ (GB)
(74) Representative: Stratagem IPM Limited

(57) **Abstract**

A device configured to detect signals indicative of a binding event in an assay of interest; the device comprising: a beam manipulator configured to modify an incident beam to form a beam with a reduced speckle pattern; a test site (18) having immobilised thereon at least one component (24) of the assay of interest; wherein the device is configured so that the beam with a reduced speckle pattern produced by the beam manipulator is incident, in use, on the test site (18) at such an angle to facilitate Total Internal Reflection (TIR); and a detector configured to receive a signal indicative of a binding event in the assay of interest; wherein the beam manipulator further comprises an optical waveguide; and wherein the optical waveguide is continuously perturbed to form a beam with a reduced speckle pattern.

## Description

### FIELD OF THE INVENTION

The present invention relates to improvements in or relating to a device for imaging, and in particular, a device for modifying a beam of light for use in imaging at least one component of an assay of interest immobilised on a test site, using Total Internal Reflection (TIR).

### BACKGROUND TO THE INVENTION

Total Internal Reflection (TIR) occurs when incident light above a critical angle impinges on an interface between a higher and lower refractive index material, producing an evanescent, exponentially decaying light field, penetrating in the lower refractive index medium. This evanescent field is restricted to the region immediately adjacent to the interface, and in typical microscopy scenarios where the light is in the visible range of the electromagnetic spectrum, the higher refractive index medium is glass and the lower refractive index medium is a liquid or cellular sample, the penetration depth is on the order of 100 nm.

TIR microscopy or spectroscopy, including cases of magnification, demagnification and 1:1 imaging, can be utilised to detect a specific analyte in a liquid sample. Capture components specific to the target analyte can be deposited directly on the TIR interface and a label bound to the target analyte allows for the detection of the analyte by producing a measurable signal within the evanescent field region. The target analyte could be a specific protein in a biological liquid sample, the capture components could be antibodies specific to the target protein, and the label could be a fluorescent or highly scattering particle.

The illumination system used in a TIR microscope requires precise optimisation to generate the best quality images. In particular, the ability to generate an illumination beam with a sharp-edged intensity cut-off can be important in some analytical applications to ensure the sample region is illuminated whilst minimising any light impinging on regions outside of the sample region. This ensures that regions within the sample contribute to the collected signal, whilst regions outside the sample do not contribute and act to reduce the purity of the signal. For example, TIR may be performed in a restricted region, such as a well or flow channel, and light hitting the edges of the region can cause complications with imaging, by producing background signal which can reduce the ability to detect binding events. Other desirable illumination beam properties include: beam shape; which can be tailored to conform to the shape of the sample or the shape of the detector, uniform intensity profile; this provides a constant illumination intensity across the sample, a suitable depth-of-focus; this should be large enough to ensure the beam is maintained with a suitably sharp-edged intensity cut-off across the length of the sample, spectral properties; the wavelength of the illumination light should be suitable to obtain the required signal from the label, intensity; the illumination intensity should be suitable to generate a useful detection signal, incident angle; the incident angle and the range of angles within the illumination beam should be sufficient to ensure TIR for the entire beam.

Additionally, for certain TIR microscope systems, particularly those that use a label based on Rayleigh scattering, the phenomenon of speckle can act to reduce the quality of the obtained images. Speckle arises from the interference of coherent wavefronts, and results in a grainy image with high-spatial frequencies, making it difficult to obtain high quality reproducible images. Therefore, methods can be employed to remove this effect, for example, by using a low or non-coherent light source.

It is against this background that the present invention has arisen.

### SUMMARY OF THE INVENTION

According to the present invention, there is provided a device configured to detect signals indicative of a binding event in an assay of interest; the device comprising: a beam manipulator configured to modify an incident beam to form a beam with a sharp-edged intensity cut-off; a test site having immobilised thereon at least one component of the assay of interest; wherein the device is configured so that the sharp-edged intensity cut-off beam produced by the beam manipulator is incident, in use, on the test site at such an angle to facilitate Total Internal Reflection (TIR); wherein the device is configured so that the sharp-edged intensity cut-off beam produced by the beam manipulator substantially conforms to the shape and size of the test site; and a detector configured to receive a signal indicative of a binding event in the assay of interest; wherein the test site is located within a microfluidic channel.

In some embodiments, there is provided a device configured to detect signals indicative of a binding event in an assay of interest; the device comprising a beam manipulator configured to modify an incident beam to form a beam with a sharp-edged intensity cut-off; a test site having immobilised thereon at least one component of the assay of interest; wherein the device is configured so that the sharp-edged intensity cut-off beam produced by the beam manipulator is incident, in use, on the test site at such an angle to facilitate Total Internal Reflection (TIR); wherein the device is configured so that the sharp-edged intensity cut-off beam produced by the beam manipulator substantially conforms to the shape and size of the test site; and a detector configured to receive a signal indicative of a binding event in the assay of interest.

The device of the present invention optimises the use of the test site by ensuring relatively uniform illumination across the test site without the deleterious effects of scatter that occurs if the beam hits the edge of the test site. The test site may be coterminous with the optical surface, or it may be configured to correspond to the size and aspect ratio of the sensor. Therefore, the array of assay spots that form the test site is matched to the sensor so that all binding events occurring anywhere on within the microarray can be detected by the sensor. Therefore, the device of the present invention optimises a beam of light for the improved detection of signals indicative of a binding event in an assay using TIR microscopy.

In the context of the present invention, the term "test site" is used to describe the site at which at least one component of an assay of interest is immobilised. In some embodiments, the components of the assay of interest may be immobilised on the test site in a microarray. In some embodiments, each component in the microarray may be a component of a different assay of interest.

The test site is located at the interface between an optically dense solid medium, and an optically less-dense liquid medium. When a sharp-edged intensity cut-off beam travels through the optically dense solid medium it undergoes total internal reflection at the test site.

The key requirement of the present invention is that the beam manipulator forms a beam with a sharp-edged intensity cut-off that is substantially co-terminus with the test site, and which is incident, in use, at the test site at such an angle to facilitate TIR. Within the context of the present invention, the term "sharp-edged intensity cut-off beam" is used to describe any beam which has a significantly sharper intensity cut-off than a Gaussian beam. This is important, since this property allows the desired regions of the sample to be illuminated whilst minimising the illumination of other regions. The sharpness parameter, *S*, defines the sharpness of the intensity cut-off. It is calculated by considering the ratio of the full-width half-maximum (FWHM) of the beam to the distance between the 10 % and 90 % intensity points of the beam. Ideally, this ratio would be as large as possible to maximise the sharpness of the intensity cut-off.

In a preferred embodiment, a beam with a sharp-edged intensity cut-off may have an intensity cut-off which is twice as sharp as a Gaussian beam. In some embodiments, the beam with a sharp-edged intensity cut-off may have an intensity cut-off which is three to four times sharper than a Gaussian beam. In some embodiments, the beam with a sharp-edged intensity cut off may have an intensity cut-off which is ten to one hundred times sharper than a Gaussian beam.

A beam of light with a sharp-edged intensity cut-off is important to create an illumination region with a sharp intensity drop-off outside the test site, ensuring the test site is sufficiently illuminated, whilst the regions outside the test site are not illuminated. This ensures regions outside the test site do not contribute to the image and consequently reduce the image quality. This is important to avoid unwanted scatter where the test site is bounded by walls or other objects. It also includes the case where objects which could produce unwanted signal lie immediately beyond the boundaries of the test site, and additionally the situation were multiple test sites are located adjacent to each other and are illuminated sequentially.

In some embodiments, the test site may have a circular geometry. In some embodiments, the test site may have a rectilinear geometry. In some embodiments, the test site may be located within a microfluidic channel. In some embodiments, in which the test site is located within a microfluidic channel, the assay may be carried out within a microfluidic device.

In a preferred embodiment, the test site has a rectilinear geometry, and the beam manipulator is configured to modify an incident beam to form a beam with a sharp-edged intensity cut-off which substantially conforms to the shape and size of the test site. Forming a beam which conforms to the shape and size of the test site enables a maximised proportion of the test site to be used to assay components of interest. For example, imaging a rectilinear test site with a Gaussian beam results in negative effects from signal scattering as the Gaussian intensity profile hits the edges of the test site structure. The device of the present invention forms a beam which better conforms to the shape and size of the test site, therefore maximising the space within the test site which can be utilised for immobilising components of an assay of interest thereon.

Preferably, the device of the present invention also comprises a beam manipulator which is configured to produce a Flat-top beam. In the context of the present invention, the term "Flat-top beam" should be understood to be any beam which has a significantly flatter intensity profile between the 10 % and 90 % intensity points of the beam compared to a Gaussian beam. A Flat-top beam is important to ensure that the intensity profile across the illumination region is relatively uniform, ensuring all regions of the test site are uniformly illuminated, and allowing the signal from different regions of the test site to be equally compared, without compromising the overall dynamic range across the test site. In some embodiments, the beam manipulator is configured to introduce a varying phase element across the beam to form a Flat-top beam.

In some embodiments, the device of the present invention may also comprise a beam manipulator which is configured to generate a collimated beam at the test site. A collimated beam at the test site ensures a small range of angles so that total internal reflection does not cause a wide range of penetration lengths for the evanescent field.

The beam manipulator of the present invention may comprise various optical arrangements to form a beam with a sharp-edged intensity discontinuity at the perimeter of the beam profile, which exists in a collimated manner, and preferably with a constant intensity across the beam cross-section.

In some embodiments, the beam manipulator may be configured to structure light from an incident beam to form a beam with a sharp-edged intensity cut-off by controlling the amplitude, the phase, or both the amplitude and the phase of a beam using various optical elements.

In some embodiments, the beam manipulator may be configured to modify the amplitude of the incident beam to form a beam with a sharp-edged intensity cut-off at the test site.

In some embodiments, the beam manipulator may comprise an optical element with an aperture. An aperture is an optical element that spatially restricts light propagation. In some embodiments, the aperture may be a hard aperture. In the context of the present invention, the term "hard aperture" is used to describe any aperture which at any spatial position transmits all the light or blocks all the light. An input beam with an arbitrary intensity profile can be shaped with a hard aperture to produce a beam with a sharp-edged intensity cut-off, as dictated by the aperture. In the embodiment in which an incident beam is shaped by a hard aperture, the incident beam may be a collimated laser beam. Although incoherent light sources can be used, coherent light sources such as lasers are more suitable due to their inherent higher brightness. In some embodiments, the aperture opening is constructed with the desired output beam shape. The input collimated laser beam may be directed towards the hard aperture, and any light that is not incident on the aperture opening may be absorbed or reflected. Light that is incident on the aperture opening is transmitted.

In some embodiments, the beam may be expanded, by any appropriate optical arrangement to minimise the divergence of the input beam incident on the aperture opening, and to increase the flatness of the portion of the beam sampled by the aperture opening. Appropriate optical arrangements include refracting telescopes, such as a Keplerian or Galilean telescope.

At a position immediately after the aperture opening, the beam exactly conforms to the shape of the aperture. In some embodiments, the aperture opening may be constructed such that immediately after the aperture opening, the beam has a sharp-edged profile, with a flatness across the beam dictated by the flatness of the portion of the beam sampled by the aperture opening. As the output beam propagates from the aperture, diffraction increases the complexity of the beam profile, and the aperture shape and sharp-edged nature of the beam is reduced as the distance from the aperture increases. In some embodiments, such as those where a circular aperture is provided, the beam profile develops diffraction rings which arise from the interaction of secondary wavelets from a plurality of point sources on the wavefront of the beam. If the aperture blocks part of the beam and thus the source of the secondary wavelets, the phase and amplitude of the remaining wavelets sum to create a different intensity profile, which evolves as the distance from the aperture increases. The resulting diffraction pattern will depend on the relative size and shape of the aperture, the distance of an observer, and the wavelength of light, and can be broadly categorized into Fresnel (near-field) or Fraunhofer (far-field) diffraction. The distance over which the beam displays an acceptable spatial profile is at least partially dictated by the quality of the input beam, the wavelength of the input beam, the size of the input beam, the divergence of the input beam, the size of the aperture, and the level of acceptability defined for the specific application.

In some embodiments, the aperture may be a soft aperture. In the context of the present invention, the term "soft aperture" is used to describe any aperture which has a transmission that varies spatially across the aperture. At a position immediately after the soft aperture, the beam intensity profile is determined by the input beam profile and the transmission profile of the soft aperture. The soft aperture can be used to modify the output beam profile to increase the sharp-edged features and improve the shape of the beam, compared to the input beam profile. An example of a soft aperture includes, but is not limited to, an Apodized Neutral Density Filter, which has a variable gradient transmission across the filter.

In some embodiments, compared to the output beam obtained using a hard aperture, the output beam obtained using a soft aperture may not be as sharp-edged. In some embodiments, the output beam produced by the soft aperture may have less severe effects of diffraction with propagation length, and the diffraction rings present with a hard aperture may be less prominent.

In some embodiments, the hard and/or soft aperture may be imaged with a suitable magnification, to further control the size, position, divergence, and propagation of the shaped output beam. In some embodiments, a significant portion of the beam energy is lost to the aperture.

In some embodiments, the beam manipulator may be configured to modify the phase of the incident beam to form a beam with a sharp-edged intensity cut-off at the test site.

The phase profile of a beam determines how that beam will propagate in space. The wavefronts of a beam are surfaces with equal optical phase. For example, curved wavefronts are associated with converging or diverging beams, flat wavefronts are associated with collimated beams, and distorted wavefronts are often indicative of a technical problem, for example damage and/or contamination on an optical element. The direction of wave propagation is perpendicular to the wavefront.

In some embodiments, the beam manipulator may be configured to impart specific phase changes across the beam profile to redistribute the beam intensity to conform to the desired intensity profile.

In some embodiments, the beam manipulator may comprise an optical element which changes the optical phase of the beam. In some embodiments, the beam manipulator may comprise multiple optical elements to modify the phase of an incident beam, such that the beam intensity profile conforms to a desired shape and sharp-edged profile. Since the optical phase elements do not physically aperture the beam, using an optical phase element to form a beam with a sharp-edged intensity cut-off is regarded as a low-loss technique. As with any optical element, losses do exist from scattering and unwanted reflections from optical surfaces.

In some embodiments, the beam manipulator may comprise one or more refractive optical elements.

In some embodiments, the phase of an incident beam can be modified using a refractive optical element. Refraction is the change of propagation direction when a wave comes from one medium into another. In some embodiments, the refractive optical element may be a specifically designed lens which can selectively modify the phase spatially across the beam, so the beam intensity is redistributed at a target plane to conform to the desired beam profile.

In some embodiments the refractive optical element can convert a collimated Gaussian input beam to a Flat-Top, sharp-edged intensity cut-off beam at a particular target plane. The distribution of intensity can be redistributed by the refractive optical element, which modifies the wavefront by imparting a specific position dependent phase change to the beam. The shape and index of the refraction of the lens determine the phase change imparted across the beam profile.

In some embodiments, the beam manipulator may comprise an additional refractive optical element. In some embodiments, the additional refractive optical element may be a specifically designed lens. In some embodiments, the additional refractive optical element may be placed at the position of the Flat-Top diverging output beam to selectively change the phase of the beam to generate a flat wavefront and thus a collimated beam at the test site. In some embodiments, the additional refractive optical element maintains the desired sharp-edged intensity distribution. The output beam generated by the first refractive optical element will naturally diffract, and the inclusion of an additional refractive optical element can significantly increase the distance over which the beam retains the desired properties.

In some embodiments, in which the beam manipulator comprises one or more refractive optical elements, the input beam may be a collimated input beam with a known intensity distribution. An example of a refractive phase element includes, but is not limited to, the πShaper from AdIOptica Optical Systems GmbH.

In some embodiments, the beam manipulator may further comprise one or more reflective optical elements. In some embodiments, the phase of an incident beam may be modified using a reflective optical element to convert a Gaussian incident beam to a Flat-Top, sharp-edged intensity cut-off beam at a particular target plane. In some embodiments, the reflective optical element may be a specifically designed mirror. In some embodiments, the beam manipulator may comprise an additional reflective optical element, which may be a specifically designed mirror. In some embodiments, the additional reflective optical element may be placed at the position of the Flat-Top diverging output beam to selectively change the phase of the beam to generate a flat wavefront and thus a collimated beam at the test site. In some embodiments, the additional reflective optical element maintains the desired sharp-edged intensity distribution. The output beam formed by the first reflective optical element will naturally diffract, and the inclusion of an additional reflective optical element can significantly increase the distance over which the beam retains the desired properties.

In some embodiments, the beam manipulator may further comprise one or more diffractive optical elements. In some embodiments, the phase of an incident beam can be modified using a diffractive optical element. Diffraction is the wave phenomenon that occurs when light interacts with a structure which imparts a position-dependent phase change. In some embodiments, the diffractive optical element may be a diffractive diffuser or may be a diffractive beam shaper.

A diffractive diffuser generates a large number of diffractive orders that overlap at a target plane. In some embodiments, in which a diffractive diffuser is used to modify the phase of an incident beam, the input beam may be collimated coherent or incoherent light. In some embodiments in which the input beam is coherent light, the phase differences of the diffractive orders are not controlled and this can lead to speckle, resulting from constructive and destructive interference of various diffraction orders. The diffractive diffuser can be designed to generate the desired sharp-edged shaped intensity profile at a specific target plane.

A diffractive beam shaper generates a large number of diffractive orders that overlap at a target plane. In some embodiments, in which a diffractive beam shaper is used to modify the phase of an incident beam, the input beam may be a high-quality Gaussian input laser beam or other coherent light source. Although incoherent light sources can be used, they are not preferred. Unlike with the diffractive diffuser, the phase difference between the diffractive orders is controlled, such that the shaped beam at the target plane can be speckle-free. In some embodiments, a diffractive beam shaper may be more sensitive to alignment sensitivity, and sensitive to intensity distortions of the incident laser beam compared to diffractive diffusers.

In some embodiments, the beam manipulator further comprises an additional diffractive element configured to collimate the beam. In some embodiments, a second diffractive element can be positioned at the target plane where the desired sharp-edged beam profile is generated, to convert the wavefronts to flat profile to create a collimated beam, which maintains the specific target shape. The beam will naturally diffract, but the inclusion of a second diffractive element can significantly increase the distance over which the beam retains the desired properties.

In some embodiments, the one or more diffractive optical elements may be transmissive. In some embodiments, the one or more diffractive optical elements may be reflective.

An alternate method which can be employed to shape the beam exploits the fact that the optical field at the focal plane of a lens or mirror is proportional to the Fourier transform of the product of the input optical field and the phase of the phase element through which the beam passes. Therefore, incorporating this concept into the beam shaping system allows a choice of lens focal length, which can modify the distance at which the desired beam shape is produced, and the dimensions and divergence of the desired beam shape.

In some embodiments, the beam manipulator may comprise an optical element configured to transform the incident beam into an Airy disc, and a focusing lens or mirror configured to produce a sharp-edged intensity cut off beam at the test site. In some embodiments, the optical element may be a phase element. In some embodiments, the optical element may be a lens or a mirror. In some embodiments, the incident beam may be converted to an Airy disc by an optical element through manipulation of the amplitude, or phase, or both the amplitude and phase of the beam. The Airy disc is an intensity distribution which consists of a bright central circle region surrounded by a series of bright and dark concentric rings. It represents the limit of diffraction, and is produced by a uniformly-illuminated (i.e., Flat-Top intensity distribution) perfect lens or mirror. This means that an aberration-free optical system does not image an object point perfectly to an image point; it is limited by diffraction.

Subsequently, the beam may be transformed into the Fourier plane and a beam with a sharp-edged intensity cut-off may be formed at the test site. The beam can be transformed into the Fourier plane by passing the beam through a focussing lens or using a mirror, after it has been transmitted through the optical element configured to transform the incident beam into an Airy disc. The Fourier transform of an Airy disc is a circular beam with a Flat-Top intensity profile. Therefore, the focussing lens or mirror can produce a Flat-Top beam at the focus.

In some embodiments, the beam manipulator may further comprise a phase element. In some embodiments, a phase element may be provided at the focal plane of the focussing lens or mirror to cancel the phase of the Flat-Top beam and produce a collimated beam at the test site. The design of such a phase element requires a knowledge of the phase of the Flat-Top beam at the focus of the lens or mirror. Thus this technique is most applicable to laser beams propagating in the fundamental Gaussian mode. Even after the collimating phase element, the beam will naturally diffract, but the inclusion of the phase element can significantly increase the distance over which the beam retains the desired properties.

In some embodiments, the beam manipulator may comprise a multimode optical waveguide and a lens adjacent to the output face of the waveguide. In some embodiments, the waveguide has an input face and an output face. Incident light may be coupled to the waveguide through the input face, and the incident beam may be shaped through propagation of light through the optical waveguide. A beam with a suitable sharp-edged intensity cut-off may be formed at the output face of the waveguide.

A waveguide is configured to form a shaped beam with a sharp-edged intensity cut-off through the provision of a plurality of layers of material of differing optical properties. The geometry of the different layers can be selected to manipulate the intensity profile of the beam. Secondary to this requirement, the use of an appropriate multimode waveguide and suitable light launching conditions can produce a beam with a uniform intensity profile. This differs from a conventional waveguide, such as a telecommunications waveguide, which will typically provide a non-uniform intensity with a higher intensity centrally and a decreasing intensity around the edges of the beam.

In some embodiments, the optical waveguide may modify an incident beam with a combination of both amplitude and phase control to form a beam with a sharp-edged intensity cut-off. A waveguide mode is a self-consistent electric field distribution. The transverse shape of the intensity distribution of the mode remains constant during propagation along the waveguide. The amplitude of the mode (i.e., gain or loss) and the phase of the mode can change during propagation, as defined by the propagation constant. The number of guided modes is dictated by wavelength, refractive index, waveguide architecture and waveguide dimensions. Each mode will experience a different refractive index, and thus the phase difference between the various modes evolves along the waveguide. The phase delay between modes dictates the interference pattern that is produced. The amplitude of each mode can evolve due to mode coupling, which is the transfer of energy from one mode to another. This mode coupling can be strongly influenced by perturbations to the waveguide, for example the specific way a fibre is bent, twisted or stretched and external sources that vibrate the fibre.

In some embodiments, a Flat-Top intensity profile can be achieved by exciting the modes within the waveguide evenly through effective mode mixing. Using an optical waveguide may redistribute the intensity of the beam by mode-mixing and avoid the losses associated with aperturing the beam.

A truly single-mode fibre will only guide the fundamental mode, as the composition and geometry of the fibre ensures this is the only mode that can exist. A multimode fibre on the other hand can support a large number of modes. In some embodiments, the waveguide may be a multimode fibre, or a light pipe, or any other suitable multimode waveguide. A light pipe works just as a multimode fibre, but is typically much shorter, thicker and often rigid. Using a light pipe to guide light can result in an increase in the amount of guided light at the output face compared to a fibre; however, the light at the output face is generally of a lower quality.

To form a beam with a sharp-edged intensity cut-off and which substantially conforms to the shape and size of the test site; the beam can be shaped by coupling light to a waveguide with a specific core shape. In some embodiments in which the beam manipulator comprises an optical waveguide, the input light may be coherent or incoherent light. Light with a low coherence may result in higher losses, because the coupling efficiency may be lower. In some embodiments, a uniform intensity profile may be formed across the beam using low coherence light, i.e., LED radiation, since low coherence light, in combination with a specific waveguide design can excite the modes in the fibre in a uniform manner. Coupling an LED, or other appropriately low coherence light source, to a multimodal waveguide, will trap a portion of light within the core of that waveguide. If the waveguide is highly multimodal, and is utilised with sufficient mode-mixing, the distribution of power in the core can be uniformly spread across the core cross-section, creating a uniform intensity profile.

In some embodiments, the multimode optical waveguide may be a square-core multimode fibre. In some embodiments, the multimode optical waveguide may have a circular geometry core. In some embodiments, non-circular core geometries, such as square or rectangular core profiles, can promote effective mode-mixing to generate a uniform intensity profile. In some embodiments, a square-core multimode fibre with a 150 µm width, may support around 50 000 modes at an illumination wavelength of 635 nm, for example. In some embodiments, in which light is coupled to a multimode waveguide, a superposition of various modes can be excited. The particular modes that are excited are dictated by the waveguide properties and the launch conditions of the incident light. For example, a low divergent incident beam can excite fewer modes than a more divergent beam.

In a preferred embodiment, the test site has a rectilinear geometry, and a rectangular-shaped fibre core is preferred which facilitates the creation of a TIR illumination profile with an aspect ratio dictated by both the aspect ratio of the rectangular fibre core and the incident angle. In a preferred embodiment, the image sensor also has a rectilinear geometry. This can allow the illumination region to better match the field-of-view of the image sensor, and thus minimise illumination light that is not contributing to the measured signal. Since the TIR surface is at an angle relative to the input beam, a square-shaped waveguide will be projected as a rectangle. Therefore, the use of an appropriately-orientated rectangular-shaped waveguide can produce a more square-shaped, or less rectangular, illumination region, which can be desirable for matching to an image sensor, which is typically rectangular with an aspect ratio of approximately 1.3. The aspect ratio is not limited to a value of 1.3 and can have a range of values. In some embodiments, the aspect ratio may be within the range of 1.2 to 1.5.

In some embodiments, in which the input light is coherent light, a granular speckle pattern may be created across the beam profile, resulting from the interference of the different supported modes. This effect is largely avoided using incoherent light, which exhibits speckle patterns with different minima and maxima for the different frequency components, which can act to cancel out the granularity to produce a smooth and homogeneous intensity distribution.

In some embodiments, the beam manipulator comprises a lens adjacent to the output face of the waveguide. At the immediate output face of the waveguide, a shaped and sharp-edged beam is produced. Once the light exits the output of the waveguide it will diffract, and consequently lose the shaping properties generated by the waveguide. The shaped beam should have a low angular spread at the test site ensuring that substantially all the light contacts the test site at an angle exceeding the critical angle. This ensures effective TIR as essentially all, or at least the vast majority, of the light is reflected. However, the divergence of the exiting beam is dictated by the numerical aperture of the fibre, and in some embodiments, this is too high for total internal reflection applications. In some embodiments, the lens may be configured to image the beam from the output face of the waveguide to create a beam of light shaped by the waveguide and with a suitably sharp-edged intensity cut-off and suitable angular propagation properties to achieve TIR at the test site. In some embodiments, the output of the waveguide can be imaged using a single lens. In some embodiments, the output of the waveguide can be imaged using a lens system to reproduce the properties of the guided light at a distance dictated by the lens arrangement.

In some embodiments, an imaging lens may magnify the output from the optical waveguide. By imaging the output face of the fibre by a magnification factor greater than unity, a larger replica of the fibre output face with a correspondingly lower divergence can be produced. The degree of magnification can be tailored to produce a shaped and sharp-edged beam with the appropriate dimensions and divergence for the application. This allows the formation of a beam with a sharp-edged intensity cut-off and desired shape at a predetermined distance from the end of the waveguide. The lens can therefore be configured to result in a beam having the desired properties at the test site.

In some embodiments, the lens may be distinguishable from the waveguide. In some embodiments, the lens may be an integral part of the waveguide. In some embodiments, the lens may be indistinguishable from the waveguide. In some embodiments the lens and the waveguide may be co-moulded pieces.

Careful consideration of the optical waveguide and lens design and lens position can achieve a beam with an appropriate depth-of-focus and dimensions at a suitable distance. The depth of focus should be sufficiently large to ensure the desired beam properties are maintained across the entire test site.

By imaging the output of the waveguide, the conditions at the output of the waveguide can be recreated, i.e., shaped and uniform intensity profile. However, TIR requires the range of angles in the input beam to be below a certain value, such that all the light experiences TIR and no light is transmitted into the test site, degrading the image quality. When imaging with a single lens, and using the thin-lens approximation, a relationship exists between object distance, s₁, image distance, s₂, and focal length, f: $\frac{1}{{s}_{1}} + \frac{1}{{s}_{2}} = \frac{1}{f}$

Here the object is the output of the waveguide, and the image is the TIR illumination region.

The focal length and position of the imaging lens determines the dimensions, position, depth-of-focus and range of angles in the imaged beam. In order to achieve a narrow angular range the imaging lens should be positioned slightly further than the focal length of the lens, producing a magnified image.

Furthermore, the magnification, M, i.e., the amount by which the image is increased relative to the object, is given by (using the thin-lens approximation): $M = - \frac{{s}_{2}}{{s}_{1}}$

The magnification will determine the size of the illumination region, and this is preferably well-matched to the test site and image sensor. Ideally the size of the image will be similar to the size of the beam at the lens, such that the beam exists in a collimated manner, ensuring a narrow angular range at the TIR surface. Furthermore, the size of the image should be well-matched to the test site and image sensor. Through a judicious choice of waveguide, lens and lens position, one can achieve a beam suitable for TIR.

In some embodiments, the low angular spread at the test site may be sufficiently low that substantially all of the light is above the critical angle for TIR. In some embodiments, the low angular spread at the test site may be sufficiently low that at least 99% of the light is above the critical angle for TIR. In some embodiments, the low angular spread at the test site may be sufficiently low that at least 75% of the light is above the critical angle for TIR.

A low angular spread at the test site is necessary for the light to meet the criteria for TIR. Incorporating TIR into the illumination system can significantly improve the ability to axially select a region of the test site that is illuminated. This selected illumination can be extremely advantageous, since the imaging system can gather signal efficiently from this illuminated thin slice, whilst non-illuminated regions of the test site do not contribute to the image. This is particularly advantageous when signal-producing objects are present beyond the axial depth of illumination on the surface of the test site, but there is a desire to restrict illumination to the surface only as restricted by the depth of the evanescent field produced by the total internal reflection process.

In an embodiment in which coherent light, for example laser radiation is input into a square-core fibre, many modes are typically excited and various mode coupling occurs along the length of the fibre due to the specific perturbation. At the output face of the fibre there is a specific combination of modes with various amplitudes which interfere to produce a speckle pattern across the beam profile defined by the specific modal interference. This speckle pattern leads to images which are grainy in nature.

In some embodiments, the beam manipulator may be configured to introduce a variable phase adjustment that varies across the beam to form a beam with a reduced speckle pattern. In some embodiments, the beam manipulator may be continuously perturbed to form a beam with a reduced speckle pattern. Continuously perturbing the system continuously varies the various modes excited and/ or the various mode coupling that exists. This can be configured such that a huge number of different speckle patterns are produced over a particular time period. When averaged over this time period, the speckle patterns can act to cancel each other out, to produce an averaged intensity profile which is significantly smoother and significantly less susceptible to specific launch conditions or other fibre perturbations. In some embodiments, the deliberate perturbation applied dominates over all other perturbations to produce a smoother beam intensity profile.

In some embodiments, the device of the present invention may further comprise a light source. In some embodiments, the light source may be an LED, or a laser, or a super luminescent diode or an amplified spontaneous emission light source.

In some embodiments, the light source may be a coherent or partially coherent light source. In some embodiments, the light source may be a laser beam. The issue of speckle is only applicable to coherent radiation such as laser beams. Non-coherent sources, such as LED radiation, do not suffer this phenomenon, since interference requires some form of phase coherence, which non-coherent sources lack.

In some embodiments, the light source may be a conventional lamp such as a halogen lamp. In some embodiments the light source may comprise a lamp coupled with a filter in order to select only relevant wavelengths. The selection of the light source will be influenced by the proportion of energy emitted in the relevant spectral region.

In some embodiments, the light source may be a singular LED. In some embodiments the light source may be multiple LEDs. The multiple LEDs may be different colours to facilitate a dual wavelength input and to broaden the spectral range.

In some embodiments, the detector may further comprise imaging optics and an imaging sensor.

In some embodiments, the imaging sensor may have a rectilinear geometry. In some embodiments, the imaging sensor may have a circular geometry. In a preferred embodiment, the assay is imaged onto a rectilinear image sensor. In some embodiments, the sharp-edged perimeter of the image sensor conforms to the shape of the rectilinear test site and the sharp-edged intensity cut-off beam. In a preferred embodiment, a rectilinear image sensor enables the creation of an aspect ratio at the test site that matches the sensor. An image sensor with an aspect ratio which conforms to the test site, enables a maximised number of components of an assay to be evenly illuminated and imaged within the finite space of the test site. Shaping a beam of light to conform to the test site is desirable to minimise light hitting undesired regions such as outside the edge of the test site, where it could scatter and negatively impact image quality. Furthermore, matching the illumination region to the square or rectangular geometry of conventional image sensors leads to an efficient architecture and maximises the use of space in the device.

Furthermore, according to the present invention, there is provided a device configured to detect signals indicative of a binding event in an assay of interest; the device comprising: a beam manipulator configured to modify an incident beam to form a beam with a reduced speckle pattern; a test site having immobilised thereon at least one component of the assay of interest; wherein the device is configured so that the beam with a reduced speckle pattern produced by the beam manipulator is incident, in use, on the test site at such an angle to facilitate Total Internal Reflection (TIR); and the device further comprising a detector configured to receive a signal indicative of a binding event in the assay of interest.

The device of the present invention may optimise the use of the test site by ensuring relatively uniform illumination across the test site without the deleterious effects of scatter that occur if the beam hits the edge of the test site. The test site may be coterminous with the optical surface, or it may be configured to correspond to the size and aspect ratio of the sensor. Therefore, the array of assay spots that form the test site is matched to the sensor so that all binding events occurring anywhere on within the microarray can be detected by the sensor. Therefore, the device of the present invention optimises a beam of light for the improved detection of signals indicative of a binding event in an assay using TIR microscopy.

In the context of the present invention, the term "test site" is used to describe the site at which at least one component of an assay of interest is immobilised. In some embodiments, the components of the assay of interest may be immobilised on the test site in a microarray. In some embodiments, each component in the microarray may be a component of a different assay of interest.

The test site is located at the interface between an optically dense solid medium, and an optically less-dense liquid medium. When a sharp-edged intensity cut-off beam travels through the optically dense solid medium it undergoes total internal reflection at the test site.

In some embodiments, the test site may have a circular geometry. In some embodiments, the test site may have a rectilinear geometry. In some embodiments, the test site may be located within a microfluidic channel. In some embodiments, in which the test site is located within a microfluidic channel, the assay may be carried out within a microfluidic device.

In some embodiments, the beam manipulator may be configured to introduce a variable phase adjustment that varies across the beam to form a beam with a reduced speckle pattern. In some embodiments, the beam manipulator may be continuously perturbed to form a beam with a reduced speckle pattern. Continuously perturbing the system continuously varies the various modes excited and/ or the various mode coupling that exists. This can be configured such that a huge number of different speckle patterns are produced over a particular time period. When averaged over this time period, the speckle patterns can act to cancel each other out, to produce an averaged intensity profile which is significantly smoother and significantly less susceptible to specific launch conditions or other fibre perturbations. In some embodiments, the deliberate perturbation applied dominates over all other perturbations to produce a smoother beam intensity profile.

In some embodiments, the beam manipulator may comprise a vibrating plate. In some embodiments, the continuous perturbation may be provided by a vibrating plate. In some embodiments, the beam manipulator may comprise a dynamic mode scrambler. In some embodiments, the effects of speckle can be reduced by a dynamic mode scrambler which temporally alters the power coupled to each mode and can act to smooth the speckle pattern when averaged over time.

In some embodiments, the beam manipulator may comprise a rotating diffuser.

In some embodiments, the beam manipulator may comprise a multimode optical waveguide and a lens to couple the light into the multimode waveguide. In some embodiments, the lens may be continuously perturbed to form a beam with a reduced speckle pattern.

In some embodiments, the beam manipulator may further comprise a diffuser plate which is continuously perturbed to form a beam with a reduced speckle pattern.

In some embodiments, speckle reduction may be achieved using other methods including, but not limited to, using piezo transducers, electric motors and electromagnets.

In some embodiments, the device of the present invention may further comprise a light source. In some embodiments, the light source may be an LED, or a laser, or a super luminescent diode or an amplified spontaneous emission light source.

In some embodiments, the light source may be a coherent or partially coherent light source. In some embodiments, the light source may be a laser beam. The issue of speckle is only applicable to coherent radiation such as laser beams. Non-coherent sources, such as LED radiation, do not suffer this phenomenon, since interference requires some form of phase coherence, which non-coherent sources lack.

In some embodiments, the light source may be a conventional lamp such as a halogen lamp. In some embodiments the light source may comprise a lamp coupled with a filter in order to select only relevant wavelengths. The selection of the light source will be influenced by the proportion of energy emitted in the relevant spectral region.

In some embodiments, the light source may be a singular LED. In some embodiments the light source may be multiple LEDs. The multiple LEDs may be different colours to facilitate a dual wavelength input and to broaden the spectral range.

In some embodiments, the detector may further comprise imaging optics and an imaging sensor.

In some embodiments, the imaging sensor may have a rectilinear geometry. In some embodiments, the imaging sensor may have a circular geometry. In a preferred embodiment, the assay is imaged onto a rectilinear image sensor. In some embodiments, the sharp-edged perimeter of the image sensor conforms to the shape of the rectilinear test site and the sharp-edged intensity cut-off beam. In a preferred embodiment, a rectilinear image sensor enables the creation of an aspect ratio at the test site that matches the sensor. An image sensor with an aspect ratio which conforms to the test site, enables a maximised number of components of an assay to be evenly illuminated and imaged within the finite space of the test site. Shaping a beam of light to conform to the test site is desirable to minimise light hitting undesired regions such as outside the edge of the test site, where it could scatter and negatively impact image quality. Furthermore, matching the illumination region to the square or rectangular geometry of conventional image sensors leads to an efficient architecture and maximises the use of space in the device.

### FIGURES

The present invention will now be described, by way of example only, with reference to the accompanying figures in which:
Figure 1 shows, schematically, a simple TIR microscope architecture;
Figure 2 shows, schematically, a TIR illumination region;
Figures 3A to 3D show various illumination scenarios suitable for achieving TIR;
Figure 4A shows a Gaussian beam profile with relevant dimensions labelled;
Figure 4B shows a beam profile for an ideal Gaussian beam with n = 2;
Figure 4C shows a beam profile for an ideal super-Gaussian beam with n = 10;
Figure 4D shows a beam profile of the measured output after conditioning with a square core fibre and an ideal Gaussian beam profile;
Figure 5A illustrates beam shaping as a Gaussian input beam is incident on a hard aperture;
Figure 5B illustrates beam shaping as a Gaussian input beam is incident on a soft aperture;
Figure 6 shows a refractive or reflective phase element shaping a collimated Gaussian input beam into a Flat-Top intensity distribution after a certain propagation distance;
Figure 7 shows a second refractive or reflective phase element modifying the phase of the diverging Flat-Top intensity distribution of Figure 6 to generate a flat wavefront;
Figure 8 shows a diffractive element converting a collimated Gaussian input beam to a Flat-Top, sharp-edged intensity cut off distribution, and a second diffractive element used to generate a collimated beam;
Figure 9 shows an optical setup used to convert a Gaussian input beam into an Airy Disc, and subsequently to produce a flat wavefront and a collimated beam;
Figure 10A shows a cross section of a multimode waveguide with a square core;
Figure 10B shows a cross section of a multimode waveguide with a rectangular core;
Figure 10C shows a cross section of a multimode waveguide with a circular core;
Figures 11A and 11B show a beam profile and corresponding central line profile for a circular core cross section fibre;
Figures 11C and 11D show a beam profile and corresponding central line profile for a square core cross section fibre;
Figures 11E and 11F show a beam profile and corresponding central line profile for a rectangular core cross section fibre;
Figure 12 illustrates the conversion of a Gaussian input beam to a flat-top, sharp-edged intensity cut-off beam at the image plane of a lens using a square-core optical fibre;
Figures 13A and 13B show illumination region images and corresponding intensity profiles achieved with laser illumination;
Figures 13C and 13D show illumination region images and corresponding intensity profiles achieved by optimising the illumination beam according to the present invention and utilising an LED light source;
Figures 14A and 14B show a TIR image of a bare auto-fluorescent substrate and an intensity profile corresponding to the cross-section;
Figure 14C shows a fluorescent micro-array on the substrate of Figure 14A, captured using the sharp-edged intensity cut-off beam shown in Figure 14C;
Figure 15A shows the intensity distribution at the output face of a square-core optical fibre when laser radiation is coupled to the fibre;
Figure 15B shows the intensity profile through the centre of the fibre of Figure 15A;
Figure 16A shows the intensity distribution at the output face of a square-core optical fibre when laser radiation is coupled to the fibre and the fibre is attached to a vibrating plate;
Figure 16B shows the intensity profile through the centre of the fibre of Figure 16A;
Figures 17A and 17B show a square core fibre imaged without and with speckle reduction, respectively and
Figures 18A and 18B show TIR images of a fluorescent micro-array without and with speckle reduction respectively.

### DETAILED DESCRIPTION OF FIGURES

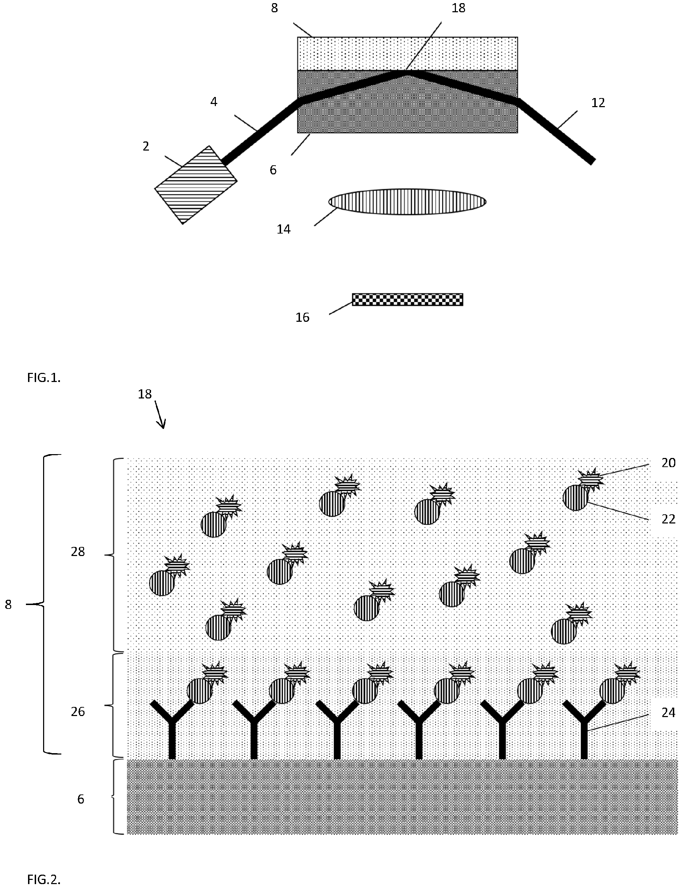

Referring to Figure 1, a simple TIR microscope architecture is shown, comprising an illumination source **2**, an input beam **4**, a higher refractive index medium **6**, a lower refractive index medium **8**, an output beam **12**, imaging optics **14** and an image sensor **16.**

TIR occurs at the interface between the higher refractive index material **6** and the lower refractive index material **8**, as the incident light above a critical angle impinges at the interface, resulting in a TIR region **18**.

At the TIR region **18**, an evanescent, exponentially decaying light field is established in the lower refractive index medium **8**. This evanescent field is restricted to the region immediately adjacent to the interface, and in typical microscopy scenarios where the higher refractive index medium **6** is glass and the lower refractive index medium **8** is a liquid sample, the penetration depth is on the order of 100 nm.

Figure 2 shows a schematic of the test site of the present invention. The test site is also the location of the TIR region **18**. Capture components of an assay of interest **24** are immobilised at the interface of the higher refractive index material **6** and the lower refractive index material **8**. The capture components **24** are therefore immobilised within the evanescent field illumination region **26** and can be utilised for the detection of a target analyte **22** in a liquid sample. In the example shown in Figure 2, the lower refractive index medium **8** is a liquid sample containing a target analyte **22** bound to a label **20.** The target analyte **22** may be a specific protein in a biological liquid sample, and the label **20** may be a fluorescent or highly scattering particle such as a nanoparticle. The capture components **24** could be antibodies specific to the target protein. The label **20** bound to the target analyte **22** allows for the detection of the target analyte **22** by producing a measurable signal. The selected illumination achieved by the limited penetration depth of the evanescent field can be extremely advantageous, since the imaging system can gather signal efficiently from the evanescent field illumination region **26,** whilst non-illuminated regions **28 of** the lower refractive index medium **8,** do not contribute to the image. Target analyte **22** located outside of the evanescent field illumination region **26** does not interfere with the image, thus achieving a high signal to background ratio. An imaging system consisting of a series of lenses or imaging optics **14** can be used to image the evanescent field illumination region **26** onto an image sensor **16.**

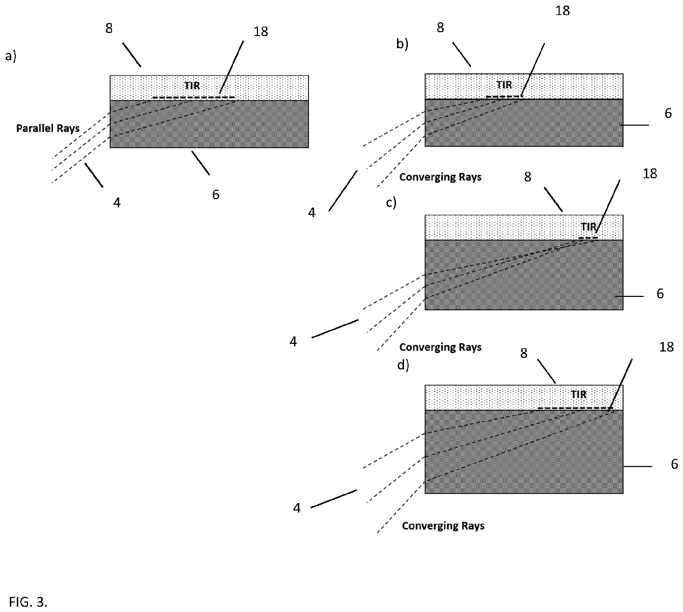

The device of the present invention optimises a beam of light for the improved detection of signals indicative of a binding event in an assay using TIR microscopy. The beam of light must satisfy the requirements for TIR at the TIR region **18.** Figure 3 shows various illumination scenarios which could achieve TIR. The incident radiation must be incident on the TIR region **18** at an angle larger than the critical angle, whereby the critical angle, ϑ*_{c}*, is defined by: ${θ}_{c} = {sin}^{- 1} \left(\frac{{n}_{2}}{{n}_{1}}\right)$ where *n₂* is the refractive index of the lower refractive index medium **8** which can be the sample material, and *n₁* is the refractive index of the higher refractive index medium **6** that the illumination beam travels through. For example, if the sample material is water, with refractive index *n₂* = 1.33, and the refractive index of the higher refractive index medium **6** is borosilicate glass, with refractive index *n₁* = 1.47, then *ϑ_{c}* = 64.8°. In this example, the illumination would need to be incident on the TIR region **18** at angles greater than 64.8°, which acts as a lower limit for the incident angle. An upper limit is naturally defined by 90°, since at this angle the light is travelling along the surface. Therefore, the range of angles that can exist in the illumination beam, *ϑ_{BEAM},* must satisfy: ${θ}_{c} < {θ}_{BEAM} < 90 °$

The range on angular tolerances depends on the geometry of the higher refractive index **6,** where the beam is incident on the higher refractive index medium **6,** the beam width, the desired geometry at the TIR region **18** and where the TIR region **18** is located. Figure 3 illustrates the substantial effect these can have on the TIR region **18.** Figure 3A shows the properties of the incident beam at the TIR region **18** with an input beam **4** with parallel rays. Figures 3B, 3C and 3D show the properties of the incident beam at the TIR region **18** with an input beam **4** with converging rays. Figures 3B, 3C and 3D also show the beam properties at the TIR region **18** with varying location of the TIR region **18,** and with varying input beam **4** width.

The key requirement of the present invention is that the beam manipulator forms a beam with a sharp-edged intensity cut-off the test site, which is incident, in use, at the test site at such an angle to facilitate TIR. The beam should have a significantly sharper intensity cut-off than a Gaussian laser beam. This is important, since this property allows the desired regions of the sample to be illuminated whilst minimising the illumination of other regions. The sharpness parameter, *S*, defines the sharpness of the intensity cut-off. It is calculated by considering the ratio of the full-width half-maximum (FWHM) of the beam to the distance between the 10 % and 90 % intensity points of the beam. Ideally, this ratio would be as large as possible to maximise the sharpness of intensity cut-off. To be effective in this context, the *S* value for the shaped beam must be at least two times greater than the *S* value for the Gaussian beam. The intensity profile of a Gaussian beam is described by: $I \left(r\right) = {I}_{0} exp \left[\left(-2{\left(\frac{r}{w}\right)}^{n}\right)\right]$ where *I₀* is the on-axis intensity, *r* is the radial position, *w* is the beam width, defined as the radial position where the intensity has reduced to *exp*(*-*2) or approximately 13.5 % of *I₀,* and *n* is the order, which is equal to 2 for a fundamental Gaussian laser beam. The above equation can be used to calculate the radial position for an arbitrary fraction of the on-axis intensity, X, given by: $r = \sqrt[n]{- \frac{{w}^{n}}{2} ln \left(X\right)}$

Using this equation and an assumed beam width of *w* = 250 µm, the radial position of the 10 % intensity point (X = 0.1) is given by 268.2 µm, and the radial position of the 90 % intensity point (X = 0.9) is given by 57.4 µm, thus the distance between these points is 210.8 µm. The radial position of the half-maximum point (X = 0.5) is given by 147.2 µm, and so the FWHM is 294.4 µm. Finally, the ratio *S* for this beam, and any beam conforming to a fundamental Gaussian, is 1.40.

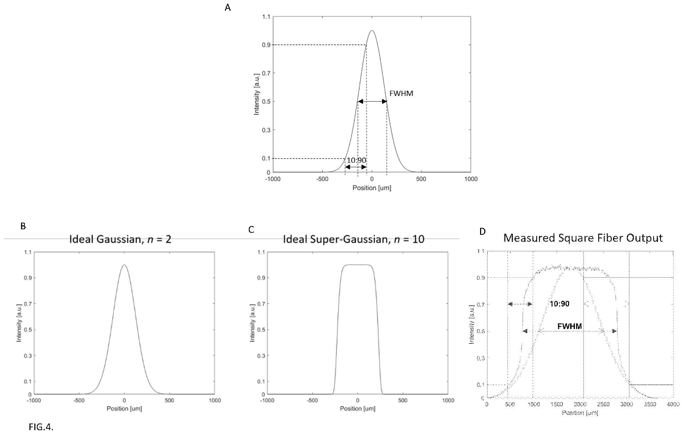

Figure 4A shows the distance between 10 % and 90 % of the on-axis intensity, and the FWHM for the example of a Gaussian beam with a 250 µm beam width. The beam profile for an ideal Gaussian beam with n=2 is shown in Figure 4B.

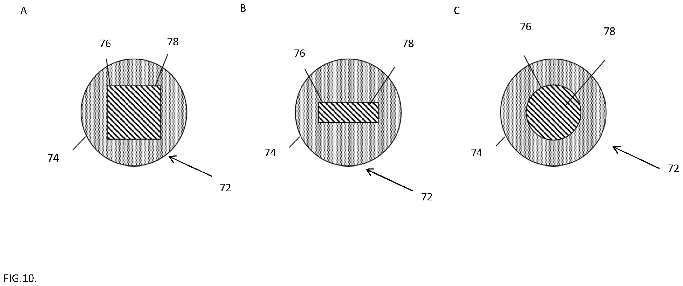

If this analysis is repeated for a super-Gaussian beam of order n = 10, a higher value of S = 6.68 is obtained. The beam profile for an ideal super-Gaussian beam with n=10 is shown in Figure 4C. The present invention facilitates the generation of a beam that exhibits a sharp-edged intensity discontinuity at the perimeter of the beam profile, which exists in a collimated manner, and with a constant intensity across the beam cross-section. The device of the present invention comprises a beam manipulator, which manipulates the phase, amplitude, or both the phase and amplitude of a beam, to produce a Flat-Top, sharp-edged beam as is also shown in Figure 4D. A value for S of approx. 4.09 was determined from the measured output after conditioning with a square core fibre shown in Figure 10A.

In a preferred embodiment, the test site may have a rectilinear geometry. By shaping the beam such that it substantially conforms to the shape and size of the test-site, interactions between the beam and test site structures can be minimised. The shaped beam can interrogate a maximised space at the test site and improve the overall image quality compared to interrogation with a Gaussian beam.

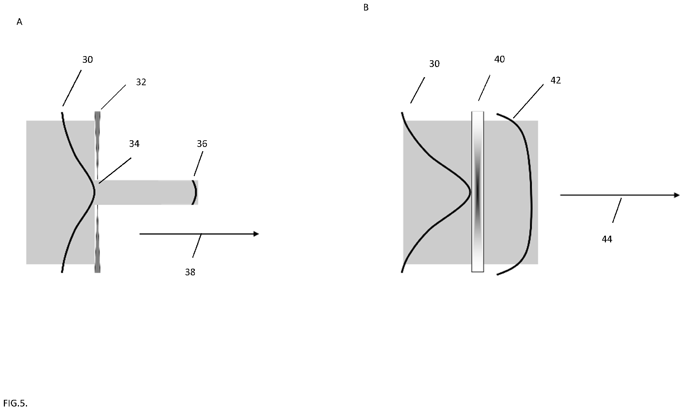

Referring to Figure 5, the beam manipulator may be configured to modify the amplitude of a Gaussian incident beam **30.** As shown in Figure 5A, the beam manipulator may be an optical element with a hard aperture **32.** The incident beam **30** is directed towards the optical element with the hard aperture **32,** and any light that is not incident on the aperture opening **34** is absorbed or reflected. The light from the incident beam **30** that is incident on the aperture opening **34** is transmitted. A shaped output beam **36** is produced.

At the position immediately after the aperture opening **34,** the shaped output beam **36** exactly conforms to the shape of the aperture opening **34.** The output beam **36** has a sharp-edged profile, with a flatness across the beam dictated by the flatness of the portion of the beam sampled by the aperture opening **34.** As the output beam **36** propagates from the aperture opening **34** in the direction shown by the arrow **38** in Figure 5A, the effects of diffraction increase the complexity of the beam profile, and the aperture shape and sharp-edged nature of the beam is reduced as the distance from the aperture increases. The distance over which the beam displays an acceptable spatial profile is at least partially dictated by the quality of the input beam **30**, the wavelength of the input beam **30**, the size of the input beam **30** the divergence of the input beam **30**, the size of the aperture opening **34**, and the level of acceptability defined for the specific application. The aperture can be imaged with a suitable magnification, to further control the size, position, divergence, and propagation of the shaped output beam **36**.

Referring to Figure 5B, the beam manipulator may be an optical element with a soft aperture **40**. The Gaussian incident beam **30** is directed towards the soft aperture **40**. The soft aperture **40** has a transmission that varies spatially across the aperture. The shape of the output beam **42** immediately after the soft aperture **40** is determined by the input beam profile and the transmission profile of the soft aperture **40**. Compared to the output beam **36** obtained using a hard aperture **32**, the output beam **42** obtained with the soft aperture **40** is not as sharp-edged. As the output beam **42** propagates from the soft aperture **40** in the direction shown by the arrow **44** in Figure 5B, the effects of diffraction increase the complexity of the beam profile. The output beam produced by the soft aperture **40** has less severe effects of diffraction with propagation length compared to the beam produced with the hard aperture **32**. The aperture can be imaged with a suitable magnification, to further control the size, position, divergence, and propagation of the shaped output beam.

The beam manipulator may be configured to modify the phase of an input beam using a refractive, reflective or diffractive optical element to form a beam with a sharp-edged intensity cut-off.

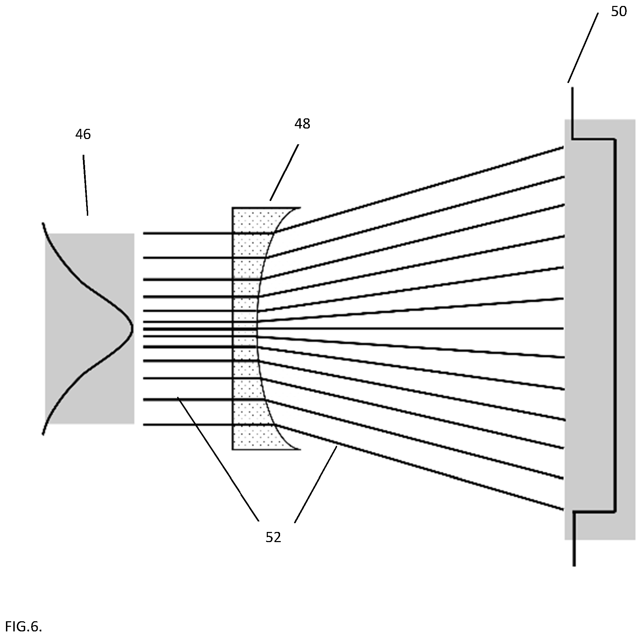

Referring to Figure 6, a collimated Gaussian input beam **46** with a known intensity distribution can be directed towards a refractive or reflective phase element **48** to convert the input beam **46** to a Flat-Top intensity distribution **50** after a certain propagation distance. The refractive or reflective phase element **48** may be a specifically designed lens or mirror. The distribution of intensity, indicated by the spacing of the rays **52**, can be redistributed by the refractive or reflective phase element **48**, which is designed to appropriately modify the wavefront, by imparting a specific position dependent phase change to the beam.

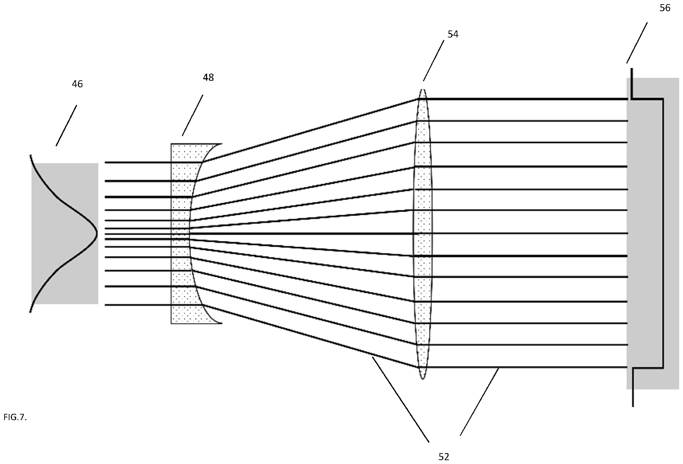

Referring to Figure 7, a second refractive or reflective phase element **54** can be placed at the position of the Flat-Top diverging output beam **50** shown in Figure 6 to selectively change the phase of the diverging Flat-Top intensity distribution to generate a flat wavefront and a shaped collimated output beam **56**. The desired sharp-edged intensity distribution is maintained. The second phase element **54** may be a specifically designed lens or mirror. The beam will naturally diffract with propagation length from the phase element **48,** but the inclusion of a second refractive or reflective phase element **54** can significantly increase the distance over which the beam retains the desired properties.

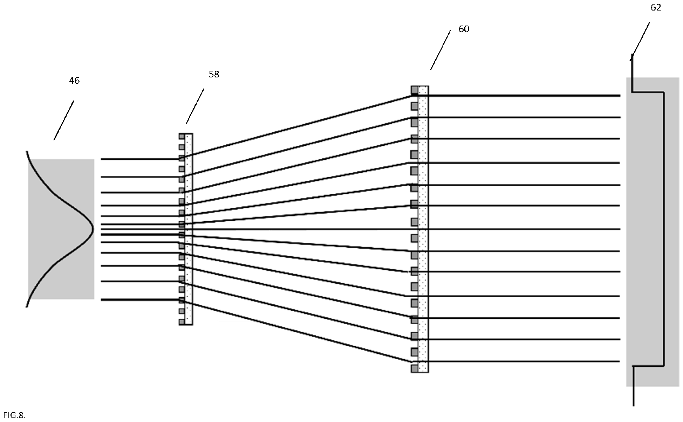

Referring to Figure 8, the beam manipulator may comprise one or more diffractive optical elements **58** and **60.** The diffractive optical elements **58** and **60** may be transmissive or reflective. The diffractive optical elements **58** and **60** may be diffractive diffusers or diffractive beam shapers. The diffractive optical element **58** modifies the phase of a collimated Gaussian input beam **46** to a Flat-Top sharp-edged intensity beam. A second diffractive element **60** located at the target plane modifies the phase of the beam to generate flat wavefronts and thus a shaped collimated output beam **62.**

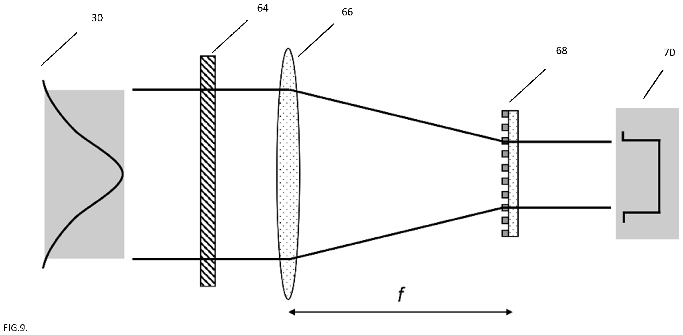

The beam manipulator may comprise an optical element configured to transform the incident beam into an Airy disc. Referring to Figure 9, an Airy disc conversion optic **64** converts the Gaussian incident beam **30** into an Airy disc and is subsequently focussed by a lens or mirror **66.** The Airy disc conversion optic **64** manipulates the amplitude, or phase, or both the amplitude and the phase of the beam. The focussing lens or mirror **66** forms the Fourier transform of the Airy disc at the focal plane, to produce a Flat-Top beam. A phase element **68** is positioned at the focal plane to modify the phase of the beam to produce flat wavefronts and a shaped collimated output beam **70.** The phase element **68** is positioned one focal length from the focussing lens or mirror **66.**

The beam manipulator may comprise an optical waveguide configured to modify an incident beam with a combination of both amplitude and phase control to form a beam with a sharp-edged intensity cut off. The optical waveguide may be a multimode optical waveguide such as a multimode fibre **72,** or may be a light pipe, or any other suitable device.

As shown in Figure 10, the multimode fibre **72** comprises a fibre cladding **74.The** cross-section of the guided light **78** will conform to that of the core **76** of the waveguide **72.** Referring to Figures 10A and 10B, preferably, the core **76** may have a rectilinear geometry. Alternatively, the core **76** may have a circular geometry as shown in Figure 10C. By utilising a waveguide with a core **76** that has a square or rectangular cross-section, guided light with low temporal coherence, uniform intensity profile and a square or rectangular cross-section can be created. The core **76** of the multimodal fibre **72** can be shaped to conform to the geometry of the test site within the evanescent field illumination region **26** and the image sensor **16.**

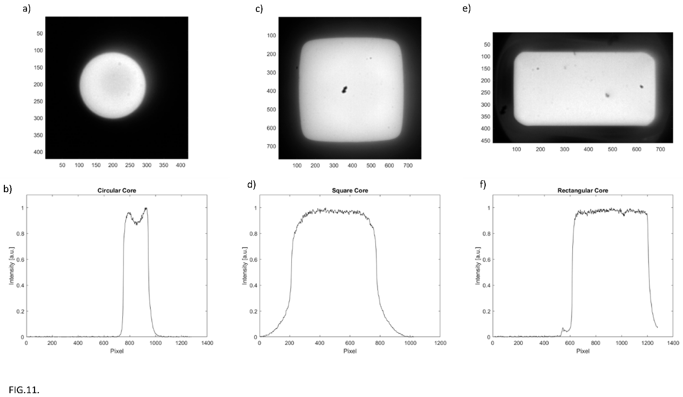

Figure 11 depicts the beam profiles and corresponding central line profiles achieved with the various core fibre cross-section geometries shown in Figures 10A to 10C. The beam profiles are taken at the image plane. Figures 11A and 11B show the beam profile and corresponding central line profile for a circular core cross-section waveguide. Figures 11C and 11D show the beam profile and corresponding central line profile for a square geometry core cross-section waveguide, and Figures 11E and 11F show the beam profile and corresponding central line profile for a rectangular geometry core cross-section waveguide. Figure 11 shows the flat-top nature of the beam is improved for the square and rectangular geometry core cross sections compared to the circular core cross section beam profile. The rectilinear geometry core cross-sections have more efficient mode mixing. The shape of the illumination region can be adapted to conform to the field of view of an image sensor. An example experimental setup used to produce the intensity profile shown in Figures 11C and 11D uses a single-colour red LED (M625L4, Thorlabs Inc.), butt-coupled to a 2 m length of square core multimode fibre with a core side length of 150 µm (M101L02, Thorlabs, Inc.). The output of which was imaged using an 8 mm focal length aspheric lens (C240TMD-B, Thorlabs, Inc.), and the subsequent image was detected at the image plane with a CMOS image sensor (DCC1645C, Thorlabs Inc.).

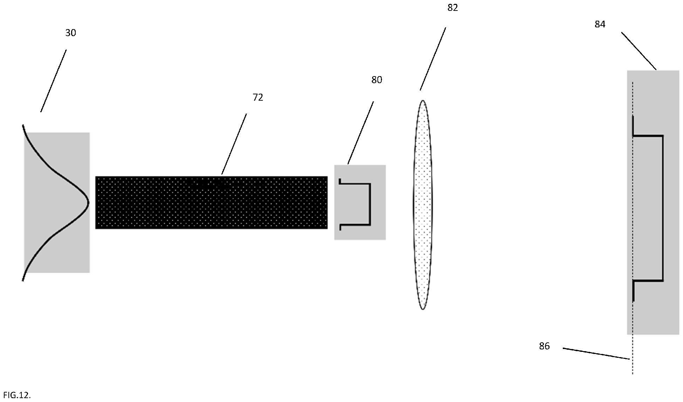

Referring to Figure 12, a Gaussian incident beam **30** is directed towards a multimode fibre **72.** As shown in Figure 12, the multimode fibre **72** may be a square-core fibre. The incident beam **30** may be coherent or incoherent light. The multimodal fibre **72** traps a portion of light within the core of the waveguide to create a guided light beam. If the waveguide is highly multimoded, the distribution of power in the core of the fibre **72** can be uniformly spread across the core cross-section, creating a uniform intensity profile. This is especially advantageous when coupled with an illumination source of low spatial coherence such as LED emission, which emits light over a wide range of angles with low directionality.

Excitation of higher-order modes with the fibre **72**, and mode-mixing between these modes leads to a Flat-Top intensity profile beam **80** at the output face of the fibre. The beam at the output of the fibre **80** is sharp-edged with a shape that conforms to the square shape of the fibre **72**. Once the light exits the output face of the multimodal waveguide **72** it will diffract, and consequently loses the properties of uniform intensity profile and square or rectangular cross-section. Therefore, an imaging lens **82** magnifies the output of the fibre **80**, to produce a Flat-Top sharp-edged beam **84** at an image plane **86**. This allows the formation of a beam of uniform intensity profile and square or rectangular cross-section at a specific distance. The Flat-Top sharp-edged beam **84** has larger dimensions than the fibre **72** and a lower divergence than the beam exiting the fibre **80**. The focal length and position of the imaging lens **82** determines the dimensions, position, depth-of-focus, and range of angles in the beam.

In order to achieve a narrow angular range the imaging lens **82** can be positioned slightly further than focal length of the imaging lens **82**, producing a magnified image. Careful consideration of the waveguide and lens design and lens position can achieve a beam with an appropriate depth-of-focus and dimensions at a suitable distance i.e., at the test site.

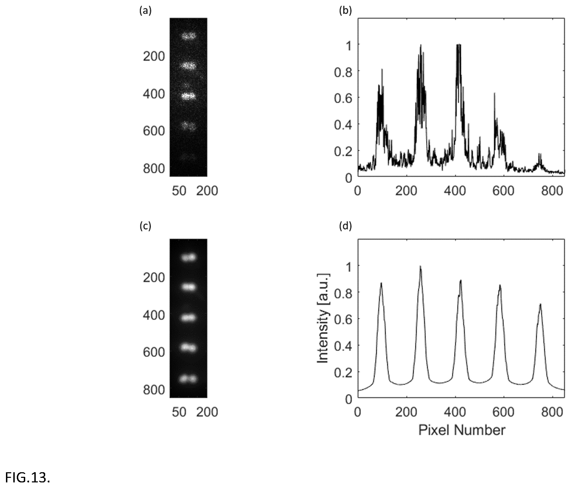

Figure 13 shows TIR images and intensity profiles from five dumbbell-shaped spots of surface-bound antibody. In this example, the sample consisted of a liquid solution which was brought into contact with a glass substrate. The sample contained a specific protein which was the target analyte. The protein could bind to the antibodies surface-bound to the glass surface, and to free antibodies in solution which were labelled with gold nanoparticles. TIR occurred at the glass/sample interface. A measurable signal was obtained when double-recognition events occurred, i.e., the protein bound both with a surface-bound antibody and a labelled mobile antibody.

Figures 13A and 13B show TIR images and intensity profiles obtained using a laser illumination source without beam shaping and a simple TIR imaging set up (as shown in Figure 1). Figure 13B shows a vertical intensity line profile through the right-hand lobe of the spots attained through laser illumination.

Figure 13C shows the images obtained from the five dumbbell-shaped spots of surface-bound antibody using the illumination architecture shown in Figure 12, utilising an LED source coupled to a square-core fibre to produce a rectangular sharp-edged intensity cut-off at the test site. Figure 13D shows a vertical intensity line profile through the right-hand lobe of the spots. The comparison between Figures 13A and 13B, and Figures 13C and 13D, clearly shows a more uniform intensity profile and significantly more even illumination distribution across the five spots attained using the illumination architecture of the present invention.

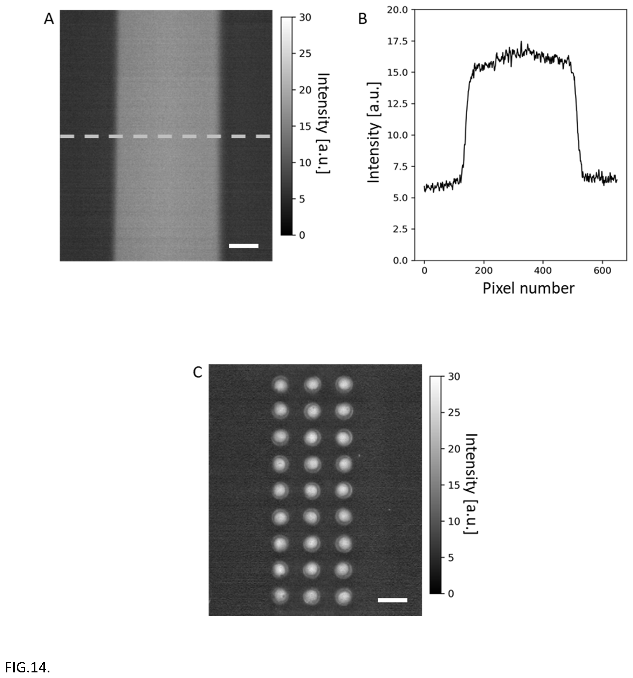

Figure 14 shows TIR images of a bare auto-fluorescent substrate with a cross-section plot and a fluorescent micro-array on a substrate with low auto-fluorescence. Figure 14A shows the auto-fluorescence from the excitation beam profile with hard-edged shaped beam. The bright vertical band in the image corresponds to the area of the substrate exposed with the beam and the dark vertical bands on the edges of the image correspond to the image background where the beam intensity is negligible. An auto-fluorescent substrate is used in this example to capture the beam profile without removing fluorescence imaging filters. The horizontal dashed line represents the location of the intensity cross-section plotted in Figure 14B. The intensity plotted is a box average of 10 nearest vertical pixels for each horizontal pixel. Figure 14C shows an image of a micro-array captured using the hard-edged shaped excitation beam. The micro-array consists of a 9 by 3 array of fluorescent spots. The spots are fluorescently labelled surface-bound antibodies. The micro-array is imaged on a higher quality substrate with lower auto-fluorescence to enhance the contrast between the spots and the image background. The scale bars shown in Figures 14A and 14C are 500 µm.

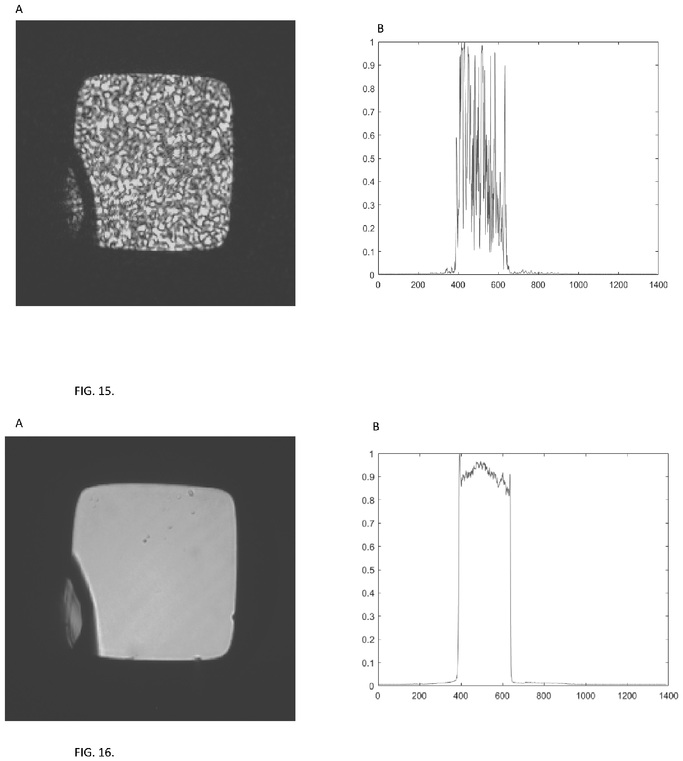

In some embodiments, in which the input light is coherent light such as laser radiation, a granular speckle pattern can be created across the beam profile, resulting from the interference of the different supported modes. Referring to Figure 15A, the intensity distribution at the output face of the square core fibre **72** of Figure 12 is shown. Referring to Figure 15B, the intensity profile through the centre of the fibre **72** is shown. The grainy nature of the image is typical of the speckle pattern.

To circumvent the issue of speckle, the fibre **72** shown in Figure 12 can be continuously perturbed. For example, the fibre **72** may be placed on a vibrating plate. The various modes excited and/or various mode coupling that exists is continuously varied and a large number of different speckle patterns are produced over a particular time period. When averaged over this time period, the speckle patterns can act to cancel each other out to produce an averaged intensity profile which is significantly smoother. Referring to Figure 16A, an image of the same fibre **72** shown in Figure 15 is shown when the fibre **72** is attached to a vibrating plate and the exposure time of the image sensor is set to one second. The effect of speckle can be seen to be minimised. Referring to Figure 16B, the intensity profile through the centre of the fibre **72** is significantly smoother. This effect is largely avoided using incoherent light such as LEDs, which exhibits speckle patterns with different minima and maxima for the different frequency components, which can act to cancel out the granularity to produce a smooth and homogeneous intensity distribution.

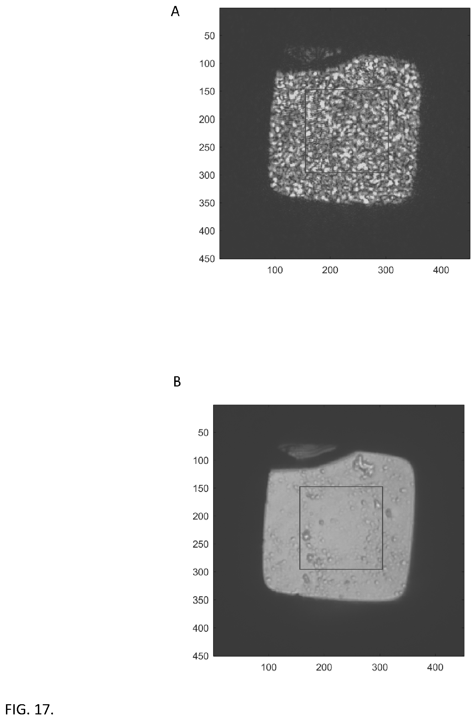

Figures 17A and 17B show a square core fibre **72** without and with speckle reduction, respectively.

In relation to quantifying an acceptable level of speckle reduction, the degree of speckle can be quantified by illuminating an image sensor with the beam of interest and determining the relative pixel intensity variations across the image. A commonly used technique for quantifying speckle in the literature is the Speckle Contrast, C, defined as: $C = {σ}_{I} / \left〈I\right〉$ where I is the pixel intensity value, <I> is the mean pixel intensity and σ_{I}, is the standard deviation of the intensity values.

A region of interest of 151 x 151 pixels is defined in both Figures 17A and 17B, by the box. The Speckle Contrast for the region of interest without speckle reduction is C = 0.658, and with speckle reduction is C = 0.087. Visually, inspecting Figures 17A and 17B there is a clear qualitative reduction in speckle and the calculation of C in each case shows a corresponding reduction in value.

The Speckle Contrast value is dependent on many factors including but not limited to: the beam size at the image sensor, background signal, the image sensor pixel size, the image sensor bit-depth and the image sensor noise properties. Specifying a particular value for C is non-trivial. However, specifying a value for C is appropriate. A value of C < 0.2 may be an appropriate target requirement.

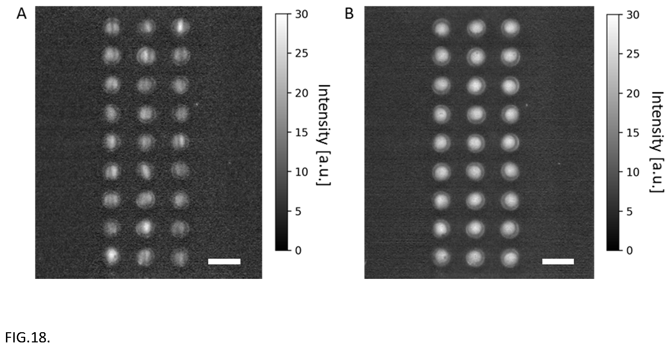

Figure 18 shows TIR images of a fluorescent micro-array with and without speckle reduction. The micro-array consists of a 9 by 3 array of fluorescent spots. The spots are fluorescently labelled surface-bound antibodies. In this example, speckle reduction was achieved using a speaker driver as the speckle reducing device and the excitation beam source was a laser coupled to a square-core multimode optical fibre **72.** The size of the excitation beam profile was matched to the size of the micro-array so that all spots in the micro-array were exposed simultaneously. The speaker driver was fixed to the optical fibre **72** such that the driver vibrations were coupled to the fibre **72** at a single point. The fibre **72** was oscillated using a square waveform at 184 Hz to reduce the observed speckle pattern on the array spots from the excitation beam.

Figure 18A shows an image of the micro-array with the speckle reduction device turned OFF and Figure 18B shows an image of the micro-array with the speckle reduction device ON. As shown in Figure 18A, when the speckle reduction device is OFF, vertical bright and dark streaks are observed on the micro-array spots. The streaks are caused by the elongation of speckle pattern at the high angle-of-incidence required for TIR imaging. The scale bars in Figures 18A and 18B are 500 µm.

Various further aspects and embodiments of the present invention will be apparent to those skilled in the art in view of the present disclosure.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

It will further be appreciated by those skilled in the art that although the invention has been described by way of example with reference to several embodiments. It is not limited to the disclosed embodiments and that alternative embodiments could be constructed without departing from the scope of the invention as defined in the appended claims.

## Claims

1. A device configured to detect signals indicative of a binding event in an assay of interest; the device comprising:
a beam manipulator configured to modify an incident beam to form a beam with a reduced speckle pattern;
a test site (18) having immobilised thereon at least one component (24) of the assay of interest;
wherein the device is configured so that the beam with a reduced speckle pattern produced by the beam manipulator is incident, in use, on the test site (18) at such an angle to facilitate Total Internal Reflection (TIR); and
a detector configured to receive a signal indicative of a binding event in the assay of interest;
wherein the beam manipulator further comprises an optical waveguide; and
wherein the optical waveguide is continuously perturbed to form a beam with a reduced speckle pattern.

2. The device according to claim 1, wherein the beam manipulator is configured to introduce a variable phase adjustment that varies across the beam to form a beam with a reduced speckle pattern.

3. The device according to any of the preceding claims, wherein the beam manipulator comprises a vibrating plate.

4. The device according to any of the preceding claims, wherein the beam manipulator comprises a dynamic mode scrambler.

5. The device according to any of the preceding claims, wherein the beam manipulator comprises a rotating diffuser.

6. The device according to any of the preceding claims, wherein the beam manipulator further comprises a multimode optical waveguide (72) and a lens (82) to couple the light into the multimode waveguide.

7. The device according to claim 6, wherein the lens (82) is continuously perturbed to form a beam with a reduced speckle pattern.

8. The device according to any of the preceding claims, wherein the beam manipulator further comprises a diffuser plate which is continuously perturbed to form a beam with a reduced speckle pattern.

9. The device according to any one of the preceding claims, further comprising a light source (2).

10. The device according to claim 9, wherein the light source (2) is a coherent or partially coherent light source.

11. The device according to claims 9 or 10, wherein the light source (2) is a laser beam.

12. The device according to any of the preceding claims, wherein the test site (18) has a rectilinear geometry.

13. The device according to any of the preceding claims, wherein the test site (18) is located within a microfluidic channel.

14. The device according to any of the preceding claims, wherein the detector further comprises imaging optics (14) and an imaging sensor (16).

15. The device according to claim 14, wherein the imaging sensor (16) has a rectilinear geometry.
